# EUROPEAN PATENT SPECIFICATION

(11) **EP 2 207 534 B1**
(45) Date of publication and mention of the grant of the patent: **09.07.2014**
(21) Application number: 08835025.1
(22) Date of filing: 03.10.2008
(51) Int. Cl.: A61K 9/20, A61K 9/28, A61K 9/50

(54) **MECHANICAL PROTECTIVE LAYER FOR SOLID DOSAGE FORMS**
MECHANISCHE SCHUTZSCHICHT FÜR FESTE DARREICHUNGSFORMEN
COUCHE DE PROTECTION MÉCANIQUE POUR FORMES DE DOSAGES SOLIDES

(30) Priority: 04.10.2007 EP 07380270
(43) Date of publication of application: 21.07.2010
(73) Proprietor: Laboratorios del. Dr. Esteve, S.A., 08041 Barcelona (ES)
(72) Inventor: SOLER RANZANI, Luis, E-08013 Barcelona (ES); CASADEVALL PUJALS, Gemma, E-08019 Barcelona (ES)
(74) Representative: ABG Patentes, S.L.
(86) International application number: PCT/EP2008/063279
(87) International publication number: WO 2009/043929

(56) References cited:
- WO-A-96/01624
- WO-A-99/59544
- WO-A-2007/020259

## Description

### FIELD OF THE INVENTION

The present invention relates to a mechanical protective layer for pellets, to a method for preparing the same and to a solid dosage form comprising the same.

### BACKGROUND OF THE INVENTION

Pharmaceutical dosage forms with different layers having different functions are used in the art. During production, solid dosage forms undergo compression processes in which some of the layers are partially damaged, and therefore require an additional protective layer or modifications in the existing layers. This is critical when said layers have functional properties and lose them after being physically damaged, and especially when said functional layer is an enteric layer, which protects the active substance from gastric fluids. If an enteric layer is damaged, the active substance may be destroyed by the highly acidic environment of the stomach.

International patent application WO 96/01624 refers to a pharmaceutical multiple unit tableted dosage form comprising an acid labile H+K+-ATPase inhibitor, in which the active substance is in the form of individually enteric coating layered units compressed into a tablet. The enteric coating layer(s) covering the individual units of active substance are plasticized, allowing that the compression of the units into a tablet does not significantly affect the acid resistance of the individually enteric coating layered units.

In WO 99/59544 and Chem. Pharm. Bull., 2003, 51(10), 1121-1127, orally disintegrable tablets having enteric coated fine granules are described. Said granules have a layer, made of a water soluble sugar-alcohol, preferably mannitol, coating the plasticized enteric layer which maybe constructed by several layers of plasticized enteric coatings.

US2005266078 describes a protective coat with at least one deformable organic constituent (e.g., a polyethylene glycol (PEG) 6000-20000) protecting a sustained release granule, said granule made by organic layering process.

WO 99/26608 addresses the problem of compression. To solve the problem it provides compressible spheroids which comprise a core covered with a flexible and deformable polymer film. The core contain at least one thermoplastic excipient with a pasty to semisolid consistency at a temperature of 20 °C allowing it to deform plastically, and thus to absorb some of the stresses to which they are subjected in a possible compression step The coat covering the core is a deformable flexible film based on a polymeric material (e.g. a PEG) with a glass transition temperature below 30 °C, which affords either protection, or masking of the taste, or controlled release of the active principle(s).

US 4684516 describe tablets containing mainly (80-100% of the tablet) sustained-release pellets coated with a retardant wax.

WO 2005120468 describes a sustained-release pellet containing a plasticized ethylcellulose layer and an external coating that protects it from erosion during the production and dosage process made of a filmogenic substance, pigments and a plasticizer. Said layer can be made of Opadry, i.e. hydroxylpropyl methylcellulose, PEG 400 and PEG 6000. Opadry is widely used as a last coat for pellets to protect them from humidity, light and also as a finish coat.

US 2002176894 describes a non enteric pharmaceutical composition which comprises a core and a drug emulsion layer, and, optionally, a protective layer, which contains PEG 20000 and is coated on the emulsified layer. Nothing is said about compression.

WO 2007/020259 discloses pharmaceutical compositions for acid-labile drugs comprising an enteric coating including a film forming agent and a mixture of triethyl citrate and cetyl alcohol as plasticizer.

### FIGURES

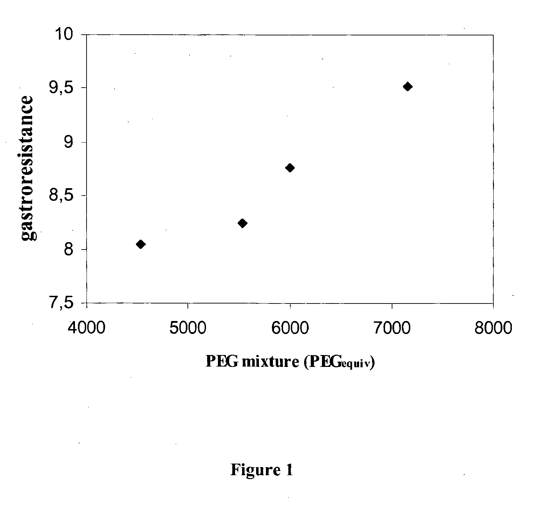

Figure 1: graphic showing the results of example 8 in the variation of gastroresistance (%) in different pellets as a function of PEG_{equiv}.

### BRIEF DESCRIPTION OF TE INVENTION

Thus, the effect of compression on enteric solid dosage forms can influence the dissolution behaviour of components and is directly related with cleavage and crushing of coating layers (e.g. enteric layers) and in consequence to its gastroresistance in acidic conditions. Therefore, there is an existing need in the art to provide a protective layer for solid dosage forms in order to prevent deterioration due to mechanical aggressions, especially compression.

According to one aspect, the present invention is directed to a mechanical protective layer for dosage forms comprising two or more plasticizer agents as defined in claim 1.

This mechanically protective layer offers advantages with respect to formulations known in the art. For example, the mechanical protective layer of the invention may be incorporated by a spray-drying processes using a highly concentrated (e.g. 27% solids) aqueous solution, allows a high amount of enteric pellets in a solid dosage form (up to 93% w/w or more in a tablet), and, thus; a high active ingredient load in a solid dosage form (e.g., 30 mg to 93.2 mg of enteric lansoprazole in 800 mg of tablet weight) and it may be used without further modification of the other layers present in a fragile solid dosage form conferring an excellent mechanical protection to said solid dosage form.

Also, the new mechanical protective layer provides tablets with high hardness, at least up to 8.5 Kp, which are not particle size dependent; pellets at least up to 0.8 mm may be compressed having the above mentioned characteristics.

According to a second aspect, the invention is directed to a method for the preparation of said mechanical protective layer which comprises dispersing all the ingredients in water and then coating the dosage form with said dispersion.

According to a third aspect, the invention is directed to a solid dosage form comprising the mechanical protective layer of the invention.

According to a fourth aspect, the invention is directed to a tablet comprising the mechanical protective layer of the invention.

### DETAILED DESCRIPTION OF THE INVENTION

As mentioned above, the mechanical protective layer for dosage forms of the invention comprises two or more plasticizer agents as defined in claim 1.

For the purposes of the invention, "mechanic protective layer" is a layer able to withstand mechanical aggression (e.g. a tabletting process) while protecting the inside contents of said layer.

For the purposes of the invention, a "plasticizer" is a substance that is normally used to improve the mechanical properties of a film formed by a polymeric substance. It is a product which does not return to its original form after deformation. When added to a polymeric substance, plasticizers provide a material with improved resistance and flexibility. For the purposes of the present invention, plasticizers are preferably solid at room temperature and water soluble.

The mechanical protective layer of the invention may comprise other substances commonly used in the art, for example, at least one additive selected from the group consisting of disintegrants, which act by swelling and/or wicking, lubricants, colorants, flavour masking agents, flavouring agents, stabilizers, binders, fillers, foaming agents, sweeteners, sweeteners, pore-forming agents, acids (e.g. citric or tartaric acid), sodium chloride, a bicarbonate (e.g. sodium or potassium), sugars and alcohols.

As examples of the masking agent, water insoluble polymers such as ethyl cellulose, polymers insoluble in saliva and soluble in gastric fluid such as a copolymer of methyl methacrylate, butyl methacrylate, and diethylaminoethyl methacrylate, and the like can be given.

By the term 'disintegrant' it is understood a substance which, upon addition to a solid preparation, facilitates its break-up or disintegration after administration and permits the release of an active ingredient as efficiently as possible to allow for its rapid dissolution. As examples of the disintegrating agent, starches such as corn starch and potato starch, partial alpha starch, sodium carboxymethyl starch, carmellose, carmellose calcium, crosscarmellose sodium, polyvinyl alcohol, crospovidone, low-substituted hydroxypropyl cellulose, crystalline cellulose, hydroxypropyl starch and the like can be given. Also, hydroxypropyl cellulose may be used as a disintegrant.

As examples of flavouring, perfume, lemon, lemon-lime, orange, menthol, peppermint oil, vanillin or powders of these absorbed with dextrin or cyclodextrin, and the like can be used.

As examples of the lubricant, magnesium stearate, magnesium stearate, fumarate stearyl, talc, stearic acid, colloidal silicon dioxide (Aerosil 200®), and the like can be given.

As examples of the colorant, food dyes such as food yellow No. 5, food red No. 3, food blue No. 2, food lake dye, red iron oxide, and the like can be given.

As examples of the stabilizer or solubilizer, antioxidants such as ascorbic acid and tocopherol, surfactants such as polysorbate 80 and the like can be given depending on the physiologically active component used.

As examples of the binder, hydroxypropyl methyl cellulose, carboxyvinyl polymer, carmellose sodium, alpha starch, polyvinylpyrrolidone, gum Arabic, gelatin, pullulan and the like can be given.

As examples of filler, sucrose, glucose, lactose, mannitol, xylitol, dextrose, microcrystalline cellulose, maltose, sorbitol, calcium phosphate, calcium sulphate and the like can be given.

As examples of the foaming agent, sodium bicarbonate can be used.

As examples of the sweetener, sodium saccharin, dipotassium glycyrrhizin, aspartame, stevia, thaumatin and the like can be given.

According to a preferred embodiment, the mechanical protective layer of the invention comprises a first plasticizer agent which is a first polyethylene glycol with an average molecular weight lower than 6000 and a second plasticizer agent which is a second polyethylene glycol, different from the first polyethylene glycol, and it comprises at least 80% w/w of plasticizer agents. Different types of polyethylene glycol are available, having different physical properties. For example, polyethylene glycol is available from a variety of providers in different average molecular weights or different densities.

According to a preferred embodiment, the average molecular weight of said first polyethylene glycol is lower than 6,000, preferably, between 3,000 and less than 6,000, more preferably, between 3,000 and 5,000.

According to a further preferred embodiment, the first polyethylene glycol has an average molecular weight between 3,000 and 5,000, more preferably of 4,000 and the second polyethylene glycol has an average molecular weight of 6,000.

According to a preferred embodiment, the mechanical protective layer of the invention comprises a third plasticizer agent which is a third polyethylene glycol, different from the first polyethylene glycol and the second polyethylene glycol.

The best results have been obtained with a mixture of PEG having different viscosity and molecular weigh. For example, as shown in the examples below, a mixture of PEG 8000, 6000 and 4000 offers excellent mechanical resistance and flexibility in a compression process and in therefore better values in gastroresistance.

Thus, according to a preferred embodiment, the mechanical protective layer of the invention comprises a first polyethylene glycol with an average molecular weight between 3,000 and 5,000, more preferably of 4,000, a second polyethylene glycol with an average molecular weight between more than 5,000 and 7,000, more preferably of 6,000 and a third polyethylene glycol with an average molecular weight between more than 7,000 and 9,000, more preferably of 8,000.

The mechanical protective layer of the invention may be used without modifying the structure of other layers present in the solid dosage form.

According to a preferred embodiment, the mechanical protective layer comprises at least 80 % w/w of a plasticizer agents, preferably at least 90% w/w of a plasticizer agents, more preferably at least 95% w/w of a plasticizer agents.

In a preferred embodiment of the invention all plasticizer agents are mixed in a single layer. However, the protective effect may also be achieved if the mechanical protective layer of the invention comprises two or more sublayers wherein each sublayer comprises one or more plasticizer agents. According to a particular embodiment, the mechanical protective layer of the invention comprises two or more sublayers wherein each sublayer comprises one plasticizer agent.

According to a further preferred embodiment, the mechanical protective layer of the invention is able to withstand mechanical aggression while protecting the inside contents of said layer in a compression (e.g. tabletting) process.

As mentioned above, a further aspect of the invention is a solid dosage form comprising the mechanical protective layer of the invention, preferably a pharmaceutically acceptable solid dosage form.

By the term 'solid dosage form' it is understood a preparation in solid state such as a tablet, granule, capsule, minitablets, fine granules, coated layers, etc.., which preferably comprises a physiologically active ingredient to be released to an appropriate medium, such as saliva, gastric fluid, water, soaps, milk, etc...

By the term "pharmaceutically acceptable solid dosage form" it is understood a preparation in solid state such as a tablet, granule, capsule, minitablets, fine granules, coated layers, etc.., which comprises a pharmaceutically active ingredient.

"Pharmaceutically acceptable" also refers to a solid dosage form that is physiologically tolerable and does not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

By the term 'gastroresistance' it is understood the amount of an active ingredient liberated after treatment for 2 hours in 0.1 N HCl at 37 °C according to the requirements of USP23 for enteric coated preparations (no individual units value exceeds 10% amount dissolved).

Said solid dosage forms comprising the mechanical protective layers of the invention have excellent gastroresistance after compression. For example, tablets with up to 93% of enteric pellet content are achieved keeping gastroresistance values under 10%.

The solid dosage forms of the invention preferably contain at least one physiologically active ingredient, which is selected from a pharmaceutically active ingredient, a flavour ingredient and a nutritional ingredient. In a particular embodiment, the solid dosage form of the invention is pharmaceutically acceptable and comprises a pharmaceutically active ingredient. In another particular embodiment, the solid dosage form is a nutritional preparation which comprises a nutritional ingredient.

As pharmaceutically active ingredient, for example, one or more ingredients selected from the group consisting of gastrointestinal function conditioning agents, anti-inflammatory agents, analgesics, anti-migraines, antihistaminic agents, cardiovascular agents, diuretics, anti-hypertensive agents, anti-hypolipidemis agents, anti-ulcer agents, anti-emetics agents, anti-asthmatic agents, anti-depressants, vitamins, anti-thrombic agents, chemotherapeutic agents, hormones, anthelmintic agents, anti-diabetic agents, anti-viral agents and mixtures thereof can be used.

Representative examples of the above-mentioned gastrointestinal function conditioning agents include bromopride, metoclopramide, cisapride and domperidone; the anti-inflammatory agents, aceclofenac, diclofenac, flubiprofen, sulindac and celecoxib; the analgesics, acetaminophen, ibuprofen and aspirin; the anti-migraines, sumatriptan and ergotamine; the antihistaminic agents, loratadine, fexofenadine and cetirizine, the cardiovascular agents, nitroglycerine, and isosorbide dinitrate; the diuretics, furocemide and spironolactone; the anti-hypertensive agents, propanolol, amlodipine, felodipine, captoprile, ramiprile, losartan, valsartan, eprosartan, irbesartan, tasosartan, telmisartan; the anti-hypolipidemic agents, simvastatin, atorvastatin and pravastatin; the anti-ulcer agents, cimetidine, ranitidine, famotidine, lansoprazole, omeprazole, rabeprazole and pantoprazole; the antiemetics, meclizine hydrochloride, ondansetron, granisetron, ramosetron and tropisetron; the anti-asthmatic agets, aminophylline, theophylline, terbutaline, fenoterol, formoterol and ketotifen; the anti-depressants, fluoxetine and sertraline; the anti-thrombotic agents, sulfinpyrazone, dipyridamole and ticlopidine; the chemotherapeutic agents, cefaclor, bacampicillin, sulfamethoxazole and rifampicin; the hormones, dexamethasone and methyltestosterone; the anthelmintic agents, pieperazine, ivermectine and mebendazole; and the anti-diabetic agents, acarbose, gliclazid and glipizid.

In a particular embodiment of the invention, the pharmaceutically active ingredient is an anti-ulcer agent or a H+/K+-ATPase inhibitor, preferably is a benzimidazole compound or one of its enantiomers or a salt thereof, more preferably is lansoprazole, omeprazole, rabeprazole or pantoprazole, even more preferably is lansoprazole.

In another particular embodiment, the pharmaceutically active ingredient is a non-steroidal anti-inflammatory drug or a salt thereof, more preferably is aspirin.

The nutritional ingredient which is included in the solid dosage form can be selected from the group consisting of vitamins, such as vitamin A, vitamin D, vitamin E (d-alpha-tocopherol acetic acid), vitamin B₁ (dibenzoyl thiamine, fursultiamine hydrochloride), vitamin B₂ (riboflavin tetrabutyrate), vitamin B₆ (pyridoxine hydrochloride), vitamin C (ascorbic acid, sodium L-ascorbate) and vitamin B₁₂ (hydroxocobalamin acetate); minerals such as calcium, magnesium and iron; proteins; amino acids; oligosaccharides, unsaturated fatty acids, herbs and mixtures thereof.

An additional advantage of the invention is that there is no need to modify the pellet structure, just to apply mechanical protective layer of the invention (or substituting a finish layer with the mechanical protective layer of the invention). For instance, the same fragile enteric pellet used in a capsule formulation, can be used also in a tablet just by coating it with the mechanical protective layer of the invention and compressing it together with a compression base (mixture) maintaining its properties and releasing profile. Additionally, the mechanical protective layer of the invention may also provide flavour masking and chemical protection without the need of further additives.

According to a preferred embodiment, the solid dosage form comprises a core comprising an inert bead coated with said mechanical protective layer.

According to a further preferred embodiment, the solid dosage form comprises a modified release layer, preferably, an enteric layer.

According to a preferred embodiment, said dosage form is a granule or pellet.

A further aspect of the invention is a tablet comprising a variable number of granules or pellets comprising the mechanical protective layer of the invention. Preferably, said tablet comprises more than 80% w/w of granules or pellets, more preferably, more than 90% w/w of granules or pellets.

According to a further aspect, the invention is directed to the use of the mechanical protective layer of the invention for the manufacture of solid dosage forms.

The mechanical protective layer of the invention may be produced following methods known in the art. According to a further aspect, the invention is directed to a method for the preparation of the mechanical protective layer of the invention, which comprises dispersing all the ingredients in water and then coating the dosage form with said dispersion.

All tablet hardness of the invention were measured with a Schleuniger Tablet Tester 8M apparatus

### EXAMPLES

### Example 1: enterically coated lansoprazole pellets without mechanical protective layer

Enterically coated lansoprazole pellets without mechanical protective layer were prepared having the composition shown in Table 1:

**Table 1**

| | | **Quantity (mg)** | **Description** |
|---|---|---|---|
| **Core** | **Inert bead** | 111.61 | Inert bead |
| | **FC 1** | 139.29 | Lansoprazole |
| | | 24.33 | Hydroxypropyl methylcellulose |
| | | 40.54 | Magnesium carbonate |
| | | 18.24 | Crospovidone |
| | | 5.27 | Talc |
| **FC 2** | | 66.84 | Hydroxypropyl methylcellulose |
| | | 8.10 | Titanium dioxide |
| | | 8.51 | Talc |
| | | 4.05 | Croscarmellose sodium |
| **FC 3** | | 106.95 | Methacrilic acid - ethyl acrylate copolymer (1:1) dispersion 30% |
| | | 15.95 | Triethyl citrate |
| | | 15.18 | Talc |

Each film coating (FC) was obtained by successively spraying different aqueous dispersions over the previous coat in a fluid bed apparatus and further drying. That is, first an inert bead of cellulose was provided. A dispersion with the components of the first film coating (FC1) was prepared and sprayed over the inert bead. Then, a dispersion of FC2 was sprayed over FC 1, and subsequently a dispersion of FC3 sprayed over FC2.

### Example 2: method for the synthesis of a mechanical protective layer of the invention

First, TEWN 80 and glyceryl monostearate were incorporated to purified water at 60-75 °C under stirring. The mixture was cooled to 25-30°C and PEG 4000, PEG 6000, PEG 8000, sodium saccharine and strawberry flavour were added. Finally, ferric oxide as red colorant was added while stirring. The dispersion so prepared is ready for coating.

### Example 3: general coating method with the mechanical protective layer of the invention

The mechanical protective layer of the invention (as prepared according to the general method of Example 2) was sprayed in a fluid bed apparatus over the enterically coated lansoprazole pellets of example 1, and further dried under the following conditions: inlet air temperature: 50-55°C, inlet air flow: 7,000-8,000 m³/h, product air temperature: 40-42°C, microclimate: 1 bar, atomizing air pressure: 3.0 bar, flow rate: 0.8-0.9 L/h

### Example 4: pellets comprising a mechanical protective layer of the invention

Following the general method of example 3, the lansoprazole pellets of example 1 were coated with a mechanical protective layer of the invention having the composition shown in Table 2.

**Table 2**

| **Quantity (mg)** | **Description** |
|---|---|
| 40.66 | Glyceryl monostearate |
| 105.71 | PEG 4000 |
| 75.22 | PEG 6000 |
| 193.94 | PEG 8000 |
| 0.31 | Ferric oxide |
| 6.10 | Saccharin sodium |
| 10.17 | Strawberry flavour |
| 3.05 | Polysorbate 80 |

### Example 5: PEG 6000 protective coat (comparative)

The pellets of example 1 were coated with PEG 6000 following the methods described in US2005266078 but using an aqueous layering process in a fluid bed apparatus. The coated pellets thus obtained were compared with the coated pellets of the invention obtained in example 4. During the spray process in a fluidized bed, the pellets of the invention obtained in example 4 showed less agglomerations than the pellets obtained in the present example.

### General method for measuring gastroresistance

The quality of the protective layers, including the new mechanical protective layer of the invention, was evaluated in examples 6 to 10 before and after tableting process by determining the amount of lansoprazole liberated after treatment for 2 h in 0.1M HCl according to the requirements of USP23 for enteric coated preparations (gastroresistance values).

In order to obtain the tablets described in Examples 6 to 10, pellets (26.93 weight percent) where mixed with a compression base in the proportions shown in Table 3 and then compressed into a tablet.

**Table 3**

| **Excipients** | % weight |
|---|---|
| Xylitol 100 | 64.28 |
| Klucel EF | 5.01 |
| Aspartame | 2.13 |
| Strawberry flavour | 0.38 |
| Masking | 0.15 |
| Red colorant | 0.01 |
| Sodium stearyl fumarate | 1.13 |
| **Total (%)** taking into account 26.93% of the pellets | 100.0 |

### Example 6: comparative - lansoprazole release

The pellets of examples 4 and 5 (26.93 weight percent) were mixed with the compression base in the proportions shown in Table 3 and compressed to tablets with final hardness of 4-6 Kp; gastroresistance of both tablets was measured.

The tablet obtained with pellets of example 4 (PEG 4000 + PEG 6000 + PEG 8000 protective coat) only increased gastroresistance values by 50-60% compared to uncompressed pellets of example 4, while the tablet obtained with pellets of example 5 (only PEG 6000 protective coat) increased gastroresistance values by 100-140% compared to uncompressed pellets of example 5. Thus, the mechanical protective layer of the invention clearly improves gastroresistance. Lower gastroresistance values indicate fewer amounts of active ingredient being released in acidic media, and therefore more efficient protection of the enteric layer.

### Example 7: influence of low molecular weight PEG

In the present example the influence of a PEG with a molecular weight below 6,000 on the plasticity of the mechanical protective layer of the invention is shown.

Following the method of example 3, the lansoprazole pellets of example 1 were coated with a mechanical protective layer of the invention having the composition shown in Table 4, to obtain pellets 7a-7d. The four mechanical layers shown in Table 4 were prepared following the same methods described in example 2. The composition of the layers only differs in the relative amounts of different PEGs. The total amount of plasticizers (PEG + glyceryl monostearate) remained essentially constant in all cases.

**Table 4**

| **Composition of the protective layer** | **Ex. 7a % weight** | **Ex. 7b % weight** | **Ex. 7c % weight** | **Ex. 7d % weight** |
|---|---|---|---|---|
| Glyceryl monostearate | 9.3 | 9.3 | 9.3 | 9.3 |
| PEG 4000 | 63.0 | 44.6 | - | - |
| PEG 6000 | 23.1 | 17.3 | 86.1 | 36.7 |
| PEG 8000 | - | 24.3 | - | 49.5 |
| Ferric oxide | 0.1 | 0.1 | 0.1 | 0.1 |
| Saccharin sodium | 1.4 | 1.4 | 1.4 | 1.4 |
| Strawberry flavour | 2.3 | 2.3 | 2.3 | 2.3 |
| Polysorbate 80 | 0.7 | 0.7 | 0.7 | 0.7 |
| **PEG**_{equiv} | **4537** | **5535** | **6000** | **7157** |
| **TOTAL** | **100.0** | **100.0** | **100.0** | **100.0** |

PEG_{equiv} was calculated as the "average" molecular weight of the PEG of the composition. For example, a 1:1 mixture of PEG 6000 and PEG 8000 would have a PEG_{equiv} of 7,000. Thus, a mixture of PEGs with a PEG_{equiv} of 7,000 is not the same as a PEG 7000.
Pellets 7a-7d (26.93 weight percent in each case) were compressed using the compression base in the proportions shown in Table 3 to a final tablet hardness of 4-6 Kp and gastroresistance was measured. The results are shown in figure 1.
As shown in figure 1, inclusion of PEGs with a molecular weight below 6,000 (e.g. PEG 4000) provides more flexibility and greater deformability during compression and, therefore, more protection for the enteric coating, which translates in better gastroresistance. Gastroresistance values increases with higher molecular weight PEGs, therefore indicating a worsening in gastroresistance.
Thus, the inclusion of PEGs with molecular weights lower than 6,000 in the plasticizer mixture seems to provide better flexibility and deformation abilities than PEG with higher molecular weights.

### Example 8: active ingredient dosage

The mechanical protective layer of the invention allows increasing the amount of pellets in a tablet, and therefore increasing active ingredient dosage, keeping tablet weight constant.
Table 5 shows 800 mg tablets containing pellets of example 1 coated with the layer of example 4, mixed with the compression base in the proportions shown in Table 3, but at different pellet proportions, and compressed to obtain a tablet (range of hardness 4-6 kp):

**Table 5**

| **Pellets (% w/w)** | **Gastroresistance** | **Lansoprazole (mg)** |
|---|---|---|
| 17 | 5.7 | 30 |
| 48.7 | 8.2 | 48.9 |
| 72.1 | 8.9 | 80.4 |
| 93 | 6 | 93.3 |

Generally, increasing the percentage of enteric pellets with regards of the compression base in a tablet increases gastroresistance values after the tableting process as pellets are crunched during compression because they are not so efficiently cushioned by the compression base. Surprisingly the mechanical protective layer of the invention allows high amounts of enteric pellets in a tablet with a low variability in gastroresistance.

### Example 9: tablet hardness

The mechanical protective layer of the invention allows the use of tablets with increased hardness (up to 8.5 Kp), therefore high compression forces may be applied, maintaining gastroresistance values constant.

Table 6 shows gastroresistance values of 800 mg tablets containing 27% pellets of example 1, coated with the protective layer of example 4 and compressed with the compression base in the proportions shown in Table 3 at different final hardness:

**Table 6**

| **Hardness (Kp)** | **Gastroresistance** |
|---|---|
| 3 | 6.1 |
| 5 | 6.2 |
| 8.5 | 6.3 |

### Example 10: pellet size

Gastroresistance of pellets with the mechanical protective layer of the invention is not size dependant. Lansoprazole pellets, enterically coated as in example 1 were coated with the protective layer of example 4. Two sizes were selected: 0.4 and 0.8 mm, and compressed to a tablet (26.93 weight percent of pellets) with the compression base in the proportions shown in Table 3 to a hardness of 4-6 kp. Gastroresistance values for both pellets size was approximately 5%.

## Claims

1. A mechanical protective layer for a dosage form comprising two or more plasticizer agents, wherein a first plasticizer agent is a first polyethylene glycol with an average molecular weight lower than 6000 and a second plasticizer agent is a second polyethylene glycol different from the first polyethylene glycol, and wherein said mechanical protective layer is present onto an enteric coated granule or pellet and wherein it comprises at least 80 % w/w of plasticizer agents.

2. The mechanical protective layer according to claim 1, comprising a first polyethylene glycol with an average molecular weight of 4,000 and a second polyethylene glycol with an average molecular weight of 6,000.

3. The mechanical protective layer according to claim 2, comprising a third plasticizer agent which is a third polyethylene glycol, different from the first polyethylene glycol and the second polyethylene glycol.

4. The mechanical protective layer according to claim 3, comprising a first polyethylene glycol with an average molecular weight of 4,000, a second polyethylene glycol with an average molecular weight of 6,000 and a third polyethylene glycol with an average molecular weight of 8,000.

5. The mechanical protective layer according to claim 1, comprising at least 90% w/w of plasticizer agents.

6. The mechanical protective layer according to claim 5, comprising at least 95% w/w of plasticizer agents.

7. The mechanical protective layer according to any of the preceding claims, wherein all plasticizer agents are mixed in a single layer.

8. The mechanical protective layer according to any claims 1 to 6, comprising two or more sublayers wherein each sublayer comprises one or more plasticizer agents.

9. The mechanical protective layer according to claim 8, comprising two or more sublayers wherein each sublayer comprises one plasticizer agent.

10. The mechanical protective layer according to any of the preceding claims, comprising a plasticizer selected from the group consisting of glyceryl monostearate, stearic acid, glyceryl palmitostearate and glyceryl dibehnate.

11. The mechanical protective layer according to any of the preceding claims, comprising at least one additive selected from the group consisting of disintegrants, which act by swelling and/or wicking; lubricants, colorants, flavours mask agents, flavouring agents, stabilizers, binders, fillers, foaming agents, sweeteners, pore-forming agents, acids, sodium chloride, a bicarbonate, sugars and alcohols.

12. A solid dosage form comprising a mechanical protective layer according to any of the preceding claims.

13. The solid dosage form of claim 12, comprising a core covered with said mechanical protective layer.

14. The solid dosage form of any of claims 12 and 13, comprising a modified release layer.

15. The solid dosage form of claim 14, wherein said modified release layer is an enteric layer.

16. The solid dosage form of any of claims 12 to 15, wherein said dosage form is a granule or a pellet.

17. A pharmaceutically acceptable solid dosage form according to any of claims 12 to 16 comprising an active ingredient.

18. A tablet comprising a variable number of granules or pellets as defined in any of claims 16 to 17.

19. The tablet of claim 18 comprising more than 80% w/w of granules or pellets.

20. The tablet of claim 19 comprising more than 90% w/w of granules or pellets.

21. Use of a mechanical protective layer comprising two or more plasticizer agents as defined in any of claims 1 to 11 for the manufacture of solid dosage forms.

22. Method for the preparation of the mechanical protective layer as defined in any of claims 1- 11, which comprises dispersing all the ingredients in water and then coating the dosage form with said dispersion.

## Patentansprüche

1. Eine mechanische Schutzschicht für eine Darreichungsform, die zwei oder mehrere Weichmacher umfasst, wobei ein erster Weichmacher ein erstes Polyethylenglykol mit einem mittleren Molekulargewicht kleiner als 6000 ist und ein zweiter Weichmacher ein zweites Polyethylenglykol ist, das von dem ersten Polyethylenglykol verschieden ist, und wobei sich die mechanische Schutzschicht auf einem magensaftresistenten Körnchen oder Kügelchen befindet und wobei sie mindestens 80 Gew.-% an Weichmachern umfasst.

2. Die mechanische Schutzschicht gemäß Anspruch 1, die ein erstes Polyethylenglykol mit einem mittleren Molekulargewicht von 4000 und ein zweites Polyethylenglykol mit einem mittleren Molekulargewicht von 6000 umfasst.

3. Die mechanische Schutzschicht gemäß Anspruch 2, die einen dritten Weichmacher umfasst, der ein drittes Polyethylenglykol ist, das von dem ersten Polyethylenglykol und dem zweiten Polyethylenglykol verschieden ist.

4. Die mechanische Schutzschicht gemäß Anspruch 3, die ein erstes Polyethylenglykol mit einem mittleren Molekulargewicht von 4000, ein zweites Polyethylenglykol mit einem mittleren Molekulargewicht von 6000 und ein drittes Polyethylenglykol mit einem mittleren Molekulargewicht von 8000 umfasst.

5. Die mechanische Schutzschicht gemäß Anspruch 1, die mindestens 90 Gew.-% an Weichmachern umfasst.

6. Die mechanische Schutzschicht gemäß Anspruch 5, die mindestens 95 Gew.-% an Weichmachern umfasst.

7. Die mechanische Schutzschicht gemäß einem der vorhergehenden Ansprüche, wobei alle Weichmacher in einer einzelnen Schicht gemischt sind.

8. Die mechanische Schutzschicht gemäß einem der Ansprüche 1 bis 6, die zwei oder mehrere Unterschichten umfasst, wobei jede Unterschicht einen oder mehrere Weichmacher umfasst.

9. Die mechanische Schutzschicht gemäß Anspruch 8, die zwei oder mehrere Unterschichten umfasst, wobei jede Unterschicht einen Weichmacher umfasst.

10. Die mechanische Schutzschicht gemäß einem der vorhergehenden Ansprüche, die einen Weichmacher, ausgewählt aus der Gruppe bestehend aus Glycerylmonostearat, Stearinsäure, Glycerylpalmitostearat und Glyceryldibehenat umfasst.

11. Die mechanische Schutzschicht gemäß einem der vorhergehenden Ansprüche, die mindestens ein Additiv umfasst, ausgewählt aus der Gruppe bestehend aus Sprengmitteln, die durch Aufquellen und/oder durch Dochtwirkung wirken; Schmiermitteln, Farbstoffen, Geschmacksmaskierungsmitteln, Aromastoffen, Stabilisatoren, Bindemitteln, Füllstoffen, Schaumbildnern, Süßstoffen, Poren bildenden Mitteln, Säuren, Natriumchlorid, einem Bicarbonat, Zuckern und Alkoholen.

12. Eine feste Darreichungsform, die eine mechanische Schutzschicht gemäß einem der vorhergehenden Ansprüche umfasst.

13. Die feste Darreichungsform gemäß Anspruch 12, die einen Kern umfasst, der mit der mechanischen Schutzschicht bedeckt ist.

14. Die feste Darreichungsform gemäß einem der Ansprüche 12 und 13, die eine modifizierte Freisetzungsschicht umfasst.

15. Die feste Darreichungsform gemäß Anspruch 14, wobei die modifizierte Freisetzungsschicht eine magensaftresistente Schicht ist.

16. Die feste Darreichungsform gemäß einem der Ansprüche 12 bis 15, wobei die Darreichungsform ein Körnchen oder ein Kügelchen ist.

17. Eine pharmazeutisch verträgliche feste Darreichungsform gemäß einem der Ansprüche 12 bis 16, die einen Wirkstoff umfasst.

18. Eine Tablette, die eine variierbare Anzahl von Körnchen oder Kügelchen, wie in einem der Ansprüche 16 bis 17 definiert, umfasst.

19. Die Tablette gemäß Anspruch 18, die mehr als 80 Gew.-% an Körnchen oder Kügelchen umfasst.

20. Die Tablette gemäß Anspruch 19, die mehr als 90 Gew.-% an Körnchen oder Kügelchen umfasst.

21. Verwendung einer mechanischen Schutzschicht, die zwei oder mehrere Weichmacher, wie in einem der Ansprüche 1 bis 11 definiert, umfasst zur Herstellung von festen Darreichungsformen.

22. Verfahren zur Herstellung der mechanischen Schutzschicht wie in einem der Ansprüche 1 bis 11 definiert, das das Dispergieren aller Inhaltsstoffe in Wasser und dann das Beschichten der Darreichungsform mit der Dispersion umfasst.

## Revendications

1. Couche de protection mécanique pour une forme galénique comprenant deux agents plastifiants ou plus, dans laquelle un premier agent plastifiant est un premier polyéthylène glycol avec une masse moléculaire moyenne inférieure à 6000 et un deuxième agent plastifiant est un deuxième polyéthylène glycol différent du premier polyéthylène glycol, et dans laquelle ladite couche de protection mécanique est présente sur un granulé ou un granulé à enrobage entérique et dans laquelle elle comprend au moins 80 % p/p d'agents plastifiants.

2. Couche de protection mécanique selon la revendication 1, comprenant un premier polyéthylène glycol avec une masse moléculaire moyenne de 4000 et un deuxième polyéthylène glycol avec une masse moléculaire moyenne de 6000.

3. Couche de protection mécanique selon la revendication 2, comprenant un troisième agent plastifiant qui est un troisième polyéthylène glycol, différent du premier polyéthylène glycol et du deuxième polyéthylène glycol.

4. Couche de protection mécanique selon la revendication 3, comprenant un premier polyéthylène glycol avec une masse moléculaire moyenne de 4000, un deuxième polyéthylène glycol avec une masse moléculaire moyenne de 6000 et un troisième polyéthylène glycol avec une masse moléculaire moyenne de 8000.

5. Couche de protection mécanique selon la revendication 1, comprenant au moins 90 % p/p d'agents plastifiants.

6. Couche de protection mécanique selon la revendication 5, comprenant au moins 95 % p/p d'agents plastifiants.

7. Couche de protection mécanique selon l'une quelconque des revendications précédentes, dans laquelle tous les agents plastifiants sont mélangés dans une couche unique.

8. Couche de protection mécanique selon l'une quelconque des revendications 1 à 6, comprenant deux sous-couches ou plus, dans laquelle chaque sous-couche comprend un ou plusieurs agents plastifiants.

9. Couche de protection mécanique selon la revendication 8, comprenant deux sous-couches ou plus, dans laquelle chaque sous-couche comprend un agent plastifiant.

10. Couche de protection mécanique selon l'une quelconque des revendications précédentes, comprenant un agent plastifiant choisi dans le groupe constitué de monostéarate de glycéryle, acide stéarique, palmitostéarate de glycéryle et dibéhénate de glycéryle.

11. Couche de protection mécanique selon l'une quelconque des revendications précédentes, comprenant au moins un additif choisi dans le groupe constitué d'agents de délitement, qui agissent par gonflement et/ou drainage ; lubrifiants, colorants, , agents masquant le goût, agents aromatisants, stabilisants, liants, agents de charge, agents moussants, édulcorants, agents formant des pores, acides, chlorure de sodium, bicarbonate, sucres et alcools.

12. Forme galénique solide comprenant une couche de protection mécanique selon l'une quelconque des revendications précédentes.

13. Forme galénique solide selon la revendication 12, comprenant un noyau couvert de ladite couche de protection mécanique.

14. Forme galénique solide selon l'une quelconque des revendications 12 et 13, comprenant une couche à libération modifiée.

15. Forme galénique solide selon la revendication 14, dans laquelle ladite couche à libération modifiée est une couche entérique.

16. Forme galénique solide selon l'une quelconque des revendications 12 à 15, dans laquelle ladite forme galénique est un granulé ou un pellet.

17. Forme galénique solide pharmaceutiquement acceptable selon l'une quelconque des revendications 12 à 16 comprenant un principe actif.

18. Comprimé comprenant un nombre variable de granulés ou de pellets tels que définis dans l'une quelconque des revendications 16 ou 17.

19. Comprimé selon la revendication 18, comprenant plus de 80 % p/p de granulés ou de pellets.

20. Comprimé selon la revendication 19, comprenant plus de 90 % p/p de granulés ou de pellets.

21. Utilisation d'une couche de protection mécanique comprenant deux agents plastifiants ou plus telle que définie dans l'une quelconque des revendications 1 à 11 pour la fabrication de formes galéniques solides.

22. Procédé de préparation de la couche de protection mécanique telle que définie dans l'une quelconque des revendications 1 à 11, qui comprend la dispersion de tous les composants dans de l'eau et ensuite l'enrobage de la forme galénique avec ladite dispersion.
